# EUROPEAN PATENT APPLICATION

(11) **EP 1 666 025 A1**
(43) Date of publication of application: **07.06.2006**
(21) Application number: 04772269.9
(22) Date of filing: 20.08.2004
(51) Int. Cl.: A61K 9/10, A61K 47/34, A61K 31/573, A61K 45/00, A61P 27/02, A61F 9/00

(54) **DRUG DELIVERY SYSTEM FOR SUB-TENON’S CAPSULE ADMINISTRATION OF FINE GRAINS**

(30) Priority: 20.08.2003 JP 2003296279
(71) Applicant: SANTEN PHARMACEUTICAL CO., LTD., Higashiyodogawa-ku, Osaka-shi, Osaka 533-8651 (JP)
(72) Inventor: YAMADA, Kazuhito Santen Pharmaceutical Co., Ltd., Osaka-shi Osaka 5338651 (JP); SASAKI, Yasumasa Santen Pharmaceutical Co., Ltd., Osaka-shi Osaka 5338651 (JP); SAKAI, Hiroyuki Santen Pharmaceutical Co., Ltd., Osaka-shi Osaka 5338651 (JP); MATSUNO, Kiyoshi Santen Pharmaceutical Co., Ltd., Osaka-shi Osaka 5338651 (JP)
(74) Representative: Peaucelle, Chantal
(86) International application number: PCT/JP2004/012313
(87) International publication number: WO 2005/018608

(57) **Abstract**

The present invention provides a drug delivery system that is a sustained drug delivery system targeting a tissue in the posterior segment of the eye accompanied by low degree of tissue invasiveness without need of frequent administration, and enables selective delivery of a drug to the posterior segment of the eye, thereby reducing influences due to transfer of the drug to the anterior segment. By administrating fine particles containing a drug to sub-Tenon, a drug delivery system enabling a drug to be selectively delivered to tissues in the posterior segment and an effective concentration to be kept can be constructed.

## Description

### Technical Field

The present invention relates to a drug delivery system targeting tissues in the posterior segment of the eye such as retina, choroid and optic nerves.

### Background Art

Many diseases in tissues in the posterior segment of the eye such as retina, choroid and optic nerves are intractable diseases, and effective therapeutic methods thereof have been desired. In therapy for ophthalmic diseases, eye drop administration of a drug has been most commonly conducted. However, the drug is hardly delivered to the tissues in the posterior segment such as retina, choroid and optic nerves. Also, even though the drug is delivered, it is very difficult to keep an effective drug concentration in the tissue.

Thus, as a method for administration of a drug for diseases in the posterior segment of the eye, intravenous injection, oral administration, intravitreal injection have been attempted. According to the intravenous injection or oral administration, quantity of the drug delivered to the tissue in the posterior segment, as a target site, is extremely slight, and in addition, undesired systemic action of the drug (side effects) may be strongly caused.

Because the intravitreal injection is a method in which a drug is directly injected to intraocular sections, quantity of the drug delivered to the tissue in the posterior segment is larger than that in the case of intravenous injection or oral administration. Drug delivery system to the posterior segment by intravitreal injection was summarized in a review (see, Journal of ocular pharmacology and therapeutics, (2001) 17/4, 393-401). However, the intravitreal injection is a method for administration which requires a high-level skill, and is accompanied by great burden on patients due to considerable pain. Thus, under current situations, intravitreal administration more than once has been very difficult also due to problems of tissue invasiveness and onset of infectious diseases.

In comparison with such intravitreal injection, sub-Tenon injection involves a comparatively simple procedure, which is accompanied by less impairment to the ocular tissues (tissue invasiveness), and is less burdensome to the patient. Sub-Tenon administration is a method conventionally employed by some clinicians. Recently, in connection with techniques relating to sub-Tenon administration, a special cannula for use in sub-Tenon administration conformed to the shape of an eyeball (see, JP-T No. 2003-511204 (the term "JP-T" as used herein means a published Japanese translation of a PCT application)) or a process for sub-Tenon implanting a capsule (see, JP-T No. 2000-507854) and the like were disclosed.

However, it is difficult to keep an effective drug concentration in tissues in the posterior segment for a long period of time, and frequent administration is required for keeping the drug concentration in the tissue. Frequent administration may increase the burden on the patient even in cases of sub-Tenon administration.

On the other hand, pharmaceutical attempts for avoiding frequent administration have been also made through keeping the intraocular drug concentration. For example, a method in which a drug-polymer conjugate is intravenously administered (see, Invest. Ophthalmol. Visual Sci. 40(11), 2690-2696, 1999), or a method in which a drug-loaded microspheres are injected to vitreous body (see, JP-A No. 2000-247871) can be exemplified, however, problems as described above have not been solved.

### Disclosure of the Invention

Accordingly, development of a sustained drug delivery system targeting a tissue in the posterior segment accompanied by low degree of tissue invasiveness without need of frequent administration has been desired. In addition, with respect to the therapy for diseases in the posterior segment, development of a drug delivery system in which a drug is selectively delivered to the posterior segment, thereby reducing influences of delivery of the drug to the anterior segment of eyes has been desired.

The present inventors elaborately made studies, and consequently found that a procedure of sub-Tenon administration of fine particles containing a drug is very useful as a drug delivery system enabling the drug to be selectively delivered to a tissue in the posterior segment and an effective concentration to be kept.

The present invention relates to a drug delivery system targeting a tissue in the posterior segment of the eye for use in sub-Tenon administration of fine particles containing a drug. The invention also relates to a preparation injectable to Tenon which is an injection comprising fine particles containing a drug and which enables drug delivery to a tissue in the posterior segment. Sub-Tenon administration of fine particles containing a drug accomplishes more satisfactory drug-deliverying capability to the tissue in the posterior segment compared to intravenous injection or oral administration, while being accompanied by less side effects. Also, the procedure is simple in comparison with intravitreal injection, and is less burdensome to the patient. Furthermore, by allowing the fine particles to contain a drug, an effective drug concentration in the target tissue can be kept for a long period of time. Moreover, high selectivity to the tissue in the posterior segment is achieved, and drug transfer to the anterior segment can be suppressed, therefore, unnecessary influence of the drug on the anterior segment is also reduced.

In the invention, material for forming the fine particle is preferably a biodegradable or biosoluble polymer, and specific examples thereof include biodegradable polymers such as polylactic acid, poly(lactic acid-glycolic acid), polylactic acid-polyethylene glycol block copolymers, polylactic acid-polyethylene glycol-polylactic acid block copolymers, poly(lactic acid-glycolic acid)-polyethylene glycol block copolymers, poly(lactic acid-glycolic acid)-polyethylene glycol-poly(lactic acid-glycolic acid) block copolymers, lactic acid-caprolactone copolymers, polyanhydride, polyortho esters, polyepsilon caprolactone, polyacrylcyano acrylate, polyhydroxy alkanoate, polyphospho esters and poly α-hydroxy acids; natural polymers such as gelatin, dextran, albumin and chitosan; synthetic polymers such as methacrylic acid copolymers and poly N-alkylacrylamide.

The molecular weight of these polymer materials is not particularly limited, but can be selected appropriately depending on type of the drug, effective therapeutic concentration of the drug and release time period of the drug, and the like.

The particle size of the fine particle in the invention is preferably 50 nm to 150 µm. Production of the fine particles having a particle size of 50 nm or less may be difficult, while the fine particles having a particle size of 150 µm or greater are so large that they are not preferred for injections. The particle size is more preferably 200 nm to 80 µm.

Examples of the fine particles in µm-order containing a drug include microspheres, while examples of the fine particles in nm-order include nanospheres.

The drug delivery system of the invention can be used for therapy or prevention for diseases of the posterior segment of the eye, in particular, of retina, choroid and optic nerves. Specific examples of the disease include inflammation resulting from various causes, viral or bacterial infectious diseases, diseases caused by retina-choroidal angiogenesis, diseases caused by retinal ischemia, optic nerve disorders caused by glaucoma. More specific examples include uveitis, cytomegalovirus retinitis, age-related macular degeneration, macular edema, diabetic retinopathy, proliferative vitreoretinopathy, retinal detachment, retinitis pigmentosa, central retinal vein occlusion, central retinal artery occlusion, and the like.

The drug included in the fine particles is not particularly limited, but any drug suited for target disease can be selected. Specific examples include steroid drugs such as betamethasone, dexamethasone, triamcinolone, prednisolone, fluorometholone, hydrocortisone and fluocinolone acetonide or derivatives thereof; hormone drugs such as progesterone and testosterone or derivatives thereof; anti-inflammatory drugs such as bromofenac and diclofenac; cytokine inhibitors such as TNF-α inhibitors, anti-TNF-α antibodies, PDE-IV inhibitors and ICE inhibitors; immunosuppressors such as cyclosporine and tacrolimus; antiviral drugs such as ganciclovir, acyclovir and interferon β; antibacterial drugs such as ofloxacin, clarithromycin and erythromycin; anticancer drugs such as fluorouracil, methotrexate and MMP inhibitors; angiogenesis inhibitors such as endostatin, VEGF inhibitors, anti-VEGF antibodies, antisense oligonucleotides, PKC inhibitors, adhesion factor inhibitors and angiostatic steroids; neuroprotective drugs and neurotrophic factors such as MK-801, timolol, creatine, taurine and BDNF, carbonate dehydratase inhibitors such as acetazolamide; thrombolytic drugs such as urokinase; circulation improving drugs, antifungal drugs and the like. As more preferable drug included in the fine particle, betamethasone, dexamethasone or fluocinolone acetonide can be exemplified.

Preferred fine particles containing a drug are matrix type prepared by dispersing a drug homogenously in the fine particle, and capsule type prepared by encapsulating a drug as a core with the fine particle.

The amount of the drug included in the fine particle can be increased or decreased appropriately depending on the type of the drug, effective therapeutic concentration, release time period of the drug, symptoms and the like. The content of the drug can be 0.01 to 95% by weight, and preferably 0.1 to 20% by weight of the fine particles.

The fine particles in the invention can be produced using a grinding process with a known mill, a phase separation process (coacervation process), a spray drying process, a super critical fluid process, an interface deposition process, or an interface reaction process, but not limited thereto. More specific processes are exemplified by a drying in liquid process that is an interface deposition process (J. Control. Release, 2, 343-352, (1985)), an interface polymerization process that is an interface reaction process (Int. J. Pharm., 28, 125-132 (1986)), a self-emulsifiable solvent diffusion process (J. Control. Release, 25, 89-98 (1993)). Among these processes for production, an appropriate process for production can be selected taking into account of the particle size of the fine particle, type, property and content of the drug, and the like.

As specific examples of the process for production of the fine particle, production examples of the fine particle containing a drug will be demonstrated in Examples described later in which betamethasone, which is an anti-inflammatory drug, was used as a drug, and polylactic acid, or poly (lactic acid-glycolic acid) was used as a material for the fine particle.

In the drug delivery system of the invention, the fine particles containing a drug are subjected to sub-Tenon administration. The method of sub-Tenon administration can be an ordinarily-conducted sub-Tenon injection. For permitting more efficient delivery of the drug to the tissue in the posterior segment, posterior sub-Tenon administration is desired. For the posterior sub-Tenon administration, a sub-Tenon's anesthesia needle can be used.

Because the fine particles which can be used in the drug delivery system of the invention are subjected to sub-Tenon administration, dosage form for administration thereof is preferably an injection. The injection solution can be prepared using a preparation technique for widely used injections. For example, the preparation can be prepared by adding commonly used additives such as e.g. , an osmoregulating agent such as sodium chloride, a buffer such as sodium phosphate, a surfactant such as polysorbate 80, a viscous agent such as methylcellulose, and the fine particles to distilled water for injection. Moreover, when a high pressure injector without a needle is utilized, the fine particles can be administered as they are without preparing an injection.

Dose of the drug can vary depending on the type of the drug, however, it is usually approximately 1 µg to 100 mg per once (the frequency can be from once or several times per day to once per several months), which can be increased or decreased depending on the age and symptoms of the patient.

Although will be described later in detail in the following Examples, in *in vitro* drug release tests, when fine particles containing betamethasone, dexamethasone and fluocinolone acetonide, respectively, were used, the drug is released in a more sustained manner than in the cases in which each powder of betamethasone, dexamethasone or fluocinolone acetonide was used. Additionally, a test carried out for measuring drug concentration in the retina-choroidal tissue ascertained the presence of the drug (betamethasone) in an effective concentration in the retina-choroidal tissue for a longer period of time in the case of sub-Tenon administration of fine particles containing betamethasone, than in the case of sub-Tenon administration of betamethasone powder. Moreover, when a test for measuring drug concentration in the aqueous humor was carried out for comparing drug concentrations in the aqueous humor in the case of sub-Tenon administration and subconjunctival administration, it was revealed that sub-Tenon administration exhibited more excellent delivering capability to the posterior retina-choroidal tissue, which is a target, also showing low delivering capability to the tissue in the anterior segment, and caused less side effects. From the foregoings, the invention characterized by sub-Tenon administration of fine particles containing a drug provides an excellent drug delivery system to a tissue in the posterior segment such as retina, choroid and optic nerves.

### Best Mode for Carrying Out the Invention

Production Examples of fine particles, *in vitro* drug release test, test for measuring drug concentration in the retina-choroidal tissue, test for measuring drug concentration in the aqueous humor, and Preparation Examples will be demonstrated below.

### 1. Production of fine particles containing a drug

### Production Example 1

Betamethasone (0.05 g) and polylactic acid (0.25 g) having a weight average molecular weight of about 20000 (degree of dispersion: about 2.0) were dissolved in dichloromethane (0.5 mL) and benzyl alcohol (3.0 mL) to give the resulting solution as a drug/polymer solution. A 0.2% (w/v) aqueous polyvinyl alcohol solution (400 mL) was homogenized with a homogenizer (10000 rpm), and thereto was added the drug/polymer solution dropwise. This mixture was homogenized for 10 min after completing the addition to prepare an O/W emulsion. This O/W emulsion was stirred using a stirrer for 3 hours (200 rpm). After completing the stirring, the resulting suspension was subjected to centrifugal separation, and the supernatant was removed. For washing the precipitates, thereto was added ultra pure water (30 mL) to disperse the precipitates, and the resulting dispersion was again subjected to centrifugal separation and the supernatant was removed. This operation was conducted once again. Particles were obtained by separating the washed precipitates with a sieve. The resulting particles were lyophilized to obtain betamethasone-loaded microspheres having a particle size of 2 µm to 70 µm, and a betamethasone content of about 12%.

### Production Example 2

Dexamethasone-loaded microspheres having a particle size of 1 µm to 80 µm, and a dexamethasone content of about 12% were obtained by carrying out a similar operation to Production Example 1 except that "dexamethasone (0.05 g)" was used in place of "betamethasone (0.05 g)" in Production Example 1.

### Production Example 3

Fluocinolone acetonide-loaded microspheres having a particle size of 3 µm to 70 µm, and a fluocinolone acetonide content of about 1% were obtained by carrying out a similar operation to Production Example 1 except that: "fluocinolone acetonide (0.05 g)" was used in place of "betamethasone (0.05 g)"; "dichloromethane (3.0 mL)" was used in place of "dichloromethane (0.5 mL) and benzyl alcohol (3.0 mL)"; and a 2.0% (w/v) aqueous polyvinyl alcohol solution was used in place of the 0.2% (w/v) aqueous polyvinyl alcohol solution in Production Example 1.

### Production Example 4

Betamethasone-loaded microspheres having a particle size of 500 nm to 70 µm, and a betamethasone content of about 12% were obtained by carrying out a similar operation to Production Example 1 except that: "poly(lactic acid-glycolic acid) (0.25 g) having a weight average molecular weight of about 20000, and a ratio lactic acid/glycolic acid of 75/25" was used in place of "polylactic acid (0.25 g) having a weight average molecular weight of about 20000 (degree of dispersion: about 2.0)"; and a 2.0% (w/v) aqueous polyvinyl alcohol solution was used in place of the 0.2% (w/v) aqueous polyvinyl alcohol solution in Production Example 1.

### 2. In vitro drug release test

1) The microspheres obtained in Production Examples 1 to 3 were charged into a chamber for *in vitro* drug release test (Spin Bio Dialyzer™ manufactured by Funakoshi Co., Ltd., having a internal capacity of 1.5 mL to which a filter having a pore size of 0.45 µm manufactured by Nihon Millipore Ltd. was attached), respectively, and thereto was added 1.5 mL of 0.1 M phosphate buffer (pH 7.4). This mixture was placed in a glass vessel, and thereto was added 98.5 mL of 0. 1M phosphate buffer (pH 7.4) . The entire mixture was shaken in a water bath at 37°C, and the *in vitro* drug release test was started. Amount of the betamethasone-loaded microspheres was determined so that the drug comes to 2.5 mg; amount of the dexamethasone-loaded microspheres was determined so that the drug comes to 3.0 mg; and amount of the fluocinolone acetonide-loaded microspheres was determined so that the drug comes to 0.5 mg, respectively. As a control, the powder of each drug in the same amount was charged into the chamber described above, and the release test was conducted in the same manner.
2) On day 1, 2, 6, 14, 29 after starting the test, the buffer was sampled in its entirety, which was analyzed using high performance liquid chromatography. Also, after the sampling, 98.5 mL of 0.1 M phosphate buffer (pH 7.4) was freshly added, and the release test was continued. Table 1 shows results of the *in vitro* drug release test.

**Table 1**

| | *In vitro* drug release rate (%) | | | | |
|---|---|---|---|---|---|
| | Day 1 | Day 2 | Day 6 | Day 14 | Day 29 |
| Betamethasone-loaded microspheres | 3.9 | 9.4 | 12.4 | 16.4 | 27.2 |
| Betamethasone powder | 51.5 | 85.0 | 97.7 | - | - |
| Dexamethasone-loaded microspheres | 9.5 | 22.0 | 29.8 | 35.0 | 43.5 |
| Dexamethasone powder | 53.6 | 73.0 | 92.1 | 96.8 | - |
| Fluocinolone acetonide-loaded microspheres | 5.5 | 12.3 | 16.1 | 23.1 | 49.6 |
| Fluocinolone acetonide powder | 57.6 | 73.4 | - | - | - |

As is apparent from Table 1, any of the microspheres (fine particles) containing betamethasone, dexamethasone or fluocinolone acetonide exhibited sustained release of the drug for a longer period of time than the powder of betamethasone, dexamethasone or fluocinolone acetonide, respectively.

### 3. Test for measuring drug concentration in the retina-choroidal tissue

The betamethasone-loaded microspheres obtained in Production Example 1 were suspended in a solvent (5% (w/v) mannitol/ 0.1% (w/v) polysorbate 80/ 0.5% (w/v) aqueous carboxymethylcellulose sodium solution) to prepare a 16.7% (w/v) injection of betamethasone-loaded microspheres. As a control, a betamethasone suspension was prepared. The betamethasone suspension was prepared by suspending betamethasone in a solvent (5% (w/v) mannitol/ 0.1% (w/v) polysorbate 80/ 0.5% (w/v) aqueous carboxymethylcellulose sodium solution) such that betamethasone concentration came to 2% (w/v).

According to the method described below, concentrations of betamethasone in the retina-choroidal tissue were measured in an animal group to which the injection of betamethasone-loaded microspheres was administered (microsphere administration group), and in an animal group to which the betamethasone suspension was administered (suspension administration group).
1) Japanese white rabbits were systemically anesthetized, and thereafter, both eyes were anesthetized on the surface by administering eye drops of oxybuprocaine hydrochloride (0.5% (w/v)).
2) The bulbar conjunctiva was incised to expose Tenon, and sub-Tenon administration of 200 µL of the 16.7% (w/v) injection of betamethasone-loaded microspheres per one-eye was conducted using a 24 G sub-Tenon's anesthesia needle. Because the content of betamethasone in the microspheres was about 12% (w/v), the dose of betamethasone came to about 4000 µg. To the suspension administration group was administered 200 µL of the 2% (w/v) betamethasone suspension per one-eye.
3) The rabbits were sacrificed 2 hours later, on day 1, 7, 14, 28, 42, 70 following the administration, and after extirpating eyeballs respectively, each retina-choroidal tissue was recovered, and the betamethasone concentration in the retina-choroidal tissue was measured by high performance liquid chromatography.

Table 2 shows results of the test for measuring drug concentration in the retina-choroidal tissue. In the Table, mean values for each 6 eyes are presented for the betamethasone concentration in the retina-choroidal tissue.

**Table 2**

| | Betamethasone concentration in retina-choroidal tissue (µg/g tissue) | |
|---|---|---|
| | Microsphere administration group | Suspension administration group (Control group) |
| 2 hrs later | 27.2 | 5.8 |
| 1 day later | 4.3 | 4.1 |
| 7 days later | 3.0 | 0.8 |
| 14 days later | 1.6 | 0.3 |
| 28 days later | 1.7 | not higher than detection limit |
| 42 days later | 1.6 | not higher than detection limit |
| 70 days later | 0.1 | not higher than detection limit |

As is apparent from Table 2, in the suspension administration group, the betamethasone concentration in the retina-choroidal tissue was about 0.3 µg/g tissue 14 days later, and was not higher than the detection limit 28 days later. In contrast, in the microspheres administration group, the betamethasone concentration in the retina-choroidal tissue was about 1.6 µg/g tissue even 42 days later, showing that the drug concentration in the retina-choroidal tissue was kept. Hence, it was revealed that the drug concentration in the retina-choroidal tissue can be kept by allowing the fine particles to contain the drug.

### 4. Test for measuring drug concentration in the retina-choroidal tissue

The betamethasone-loaded microspheres obtained in Production Example 4 were suspended in a solvent (aqueous solution of 0.4% (w/v) polysorbate 80/ 2.6% (w/v) glycerin) to prepare a 10% (w/v) injection of betamethasone-loaded microspheres. After posterior sub-Tenon administration using this injection of betamethasone-loaded microspheres according to the method described below, concentrations in the anterior and posterior retina-choroidal tissues of betamethasone were measured. As a control, measurement of the concentration after subconjunctival administration using the aforementioned injection of betamethasone-loaded microspheres was carried out, and the concentrations of betamethasone in the retina-choroidal tissue were compared with respect to the posterior sub-Tenon administration group and the subconjunctival administration group.
1) Japanese white rabbits were systemically anesthetized, and thereafter, both eyes were anesthetized on the surface by administering eye drops of oxybuprocaine hydrochloride (0.5% (w/v)).
2) The bulbar conjunctiva was incised to expose Tenon, and sub-Tenon administration of 100 µL of the injection of betamethasone-loaded microspheres per one-eye was conducted using a 24 G sub-Tenon's anesthesia needle. Because the content of betamethasone in the microspheres was about 12% (w/v), the dose of betamethasone came to about 1200 µg. To the control group was administered 100 µL of the 10% (w/v) injection of betamethasone-loaded microspheres per one-eye using a syringe with a 27 G needle to the upper part of the subconjunctiva.
3) The rabbits were sacrificed on day 7 following the administration, and after extirpating the eyeballs respectively, the anterior and posterior retina-choroidal tissues were recovered, and each betamethasone concentration in the anterior and posterior retina-choroidal tissues was measured by high performance liquid chromatography.

Table 3 shows results of measuring drug concentration in the retina-choroidal tissue. In the Table, mean values for each 3 or 4 eyes are presented for the betamethasone concentration in the retina-choroidal tissue.

**Table 3**

| | Betamethasone concentration in retina-choroidal tissue (µg/g tissue) | |
|---|---|---|
| | Posterior sub-Tenon administration | Subconjunctival administration (Control group) |
| Anterior retina-choroidal tissue | not higher than detection limit | 0.6 |
| Posterior retina-choroidal tissue | 1.6 | 1.2 |

As is apparent from Table 3, according to the subconjunctival administration, the betamethasone concentration in the anterior retina-choroidal tissue was about 0.6 µg/g tissue on day 7 after the administration, while the betamethasone concentration in the posterior retina-choroidal tissue was about 1.2 µg/g tissue. In contrast, according to the posterior sub-Tenon administration, the betamethasone concentration in the anterior retina-choroidal tissue was not higher than the detection limit on day 7 after the administration, while the betamethasone concentration in the posterior retina-choroidal tissue was about 1.6 µg/g tissue. Hence, betamethasone was delivered selectively to the posterior retina-choroidal tissue. Accordingly, it was revealed that in comparison with subconjunctival administration, sub-Tenon administration achieved more efficient delivery of the drug to the posterior retina-choroidal tissue which is particularly targeted in the choroid.

### 5. Test for measuring drug concentration in the aqueous humor

The betamethasone-loaded microspheres obtained in Production Example 4 were suspended in a solvent (aqueous solution of 0.4% (w/v) polysorbate 80/ 2.6% (w/v) glycerin) to prepare a 10% (w/v) injection of microspheres containing betamethasone. Posterior sub-Tenon administration was carried out using this injection of betamethasone-loaded microspheres according to the method described above, and concentration of betamethasone in the aqueous humor following the administration was measured. As a control, measurement of the concentration after subconjunctival administration using the aforementioned injection of betamethasone-loaded microspheres was carried out, and the concentrations of betamethasone in the aqueous humor were compared with respect to the posterior sub-Tenon administration group and the subconjunctival administration group. The rabbits were sacrificed in 1, 2, 4 hours following the administration, and each aqueous humor was recovered respectively. The betamethasone concentration in the aqueous humor was measured by high performance liquid chromatography.

Table 4 shows results of the test for measuring drug concentration in the aqueous humor. In the Table, mean values for each 4 eyes are presented for the betamethasone concentration in the aqueous humor.

**Table 4**

| | Betamethasone concentration in aqueous humor (µg/mL) | |
|---|---|---|
| | Posterior sub-Tenon administration | Subconjunctival administration (Control group) |
| 1 hour later | not higher than detection limit | 0.05 |
| 2 hours later | not higher than detection limit | 0.10 |
| 4 hours later | not higher than detection limit | 0.21 |

As is apparent from Table 4, according to the subconjunctival administration, the concentration was about 0.05, 0.10, and 0.21 µg/mL in 1, 2, 4 hours following the administration. In contrast, according to the posterior sub-Tenon administration, the concentration was not higher than the detection limit until 1 to 4 hours later, exhibiting delivering capability of betamethasone to the anterior segment lower than in the case of the subconjunctival administration. Therefore, sub-Tenon administration can reduce side effects such as increase in intraocular pressure, compared to the subconjunctival administration.

### 6. Preparation Example

| Injection 1 (100 ml) | |
|---|---|
| Betamethasone-loaded microspheres | 16.7 g |
| Mannitol | 5 g |
| Polysorbate 80 | 0.1 g |
| Carboxymethylcellulose sodium | 0.5 g |
| Sterile purified water | q.s. |
| | 100 ml |

| Injection 2 (100 ml) | |
|---|---|
| Betamethasone-loaded microspheres | 10.0g |
| Conc. glycerin | 2.6 g |
| Polysorbate 80 | 0.4 g |
| Sterile purified water | q.s. |
| | 100 ml |

### Industrial Applicability

According to the present invention, a drug delivery system enabling a drug to be selectively delivered to tissues in the posterior segment of the eye and an effective concentration to be kept can be constructed by sub-Tenon administration of fine particles containing the drug.

## Claims

1. A drug delivery system targeting a tissue in the posterior segment of the eye which comprises administrating fine particles containing a drug to sub-Tenon.

2. An injection solution for sub-Tenon administration which comprises fine particles containing a drug, and enables the drug to be selectively delivered to a tissue in the posterior segment of the eye and an effective concentration of the drug to be kept.

3. The drug delivery system according to claim 1 or the injection solution for sub-Tenon administration according to claim 2 wherein the fine particle has a mean particle size of 50 nm to 150 µm.

4. The drug delivery system according to claim 1 or the injection solution for sub-Tenon administration according to claim 2 wherein the fine particle is made of a biodegradable or biosoluble polymer.

5. The drug delivery system according to claim 1 or the injection solution for sub-Tenon administration according to claim 2 wherein the tissue in the posterior segment of the eye is retina, choroid or an optic nerve.

6. The drug delivery system according to claim 1 or the injection solution for sub-Tenon administration according to claim 2 wherein the drug is for use in therapy and/or prevention for a retinal, choroidal or optic nerve disease.

7. The drug delivery system according to claim 1 or the injection solution for sub-Tenon administration according to claim 2 wherein the drug is an anti-inflammatory drug, an immunosuppressor, an antiviral drug, an anticancer drug, a angiogenesis inhibitor, an antithrombotic drug, an optic neuroprotective drug, a circulation improving drug, an antibacterial drug or an antifungal drug.

8. The drug delivery system according to claim 1 or the injection solution for sub-Tenon administration according to claim 2 wherein the drug is a steroid.

9. The drug delivery system according to claim 1 or the injection solution for sub-Tenon administration according to claim 2 wherein the drug is betamethasone, dexamethasone or fluocinolone acetonide.

10. A method of therapy and/or prevention for a disease of a tissue in the posterior segment which comprises carrying out sub-Tenon administration of an injection solution comprising fine particles containing a drug to a patient in a therapeutic effective amount.

11. The method of therapy and/or prevention for a disease of a tissue in the posterior segment according to claim 10 wherein the fine particle has a mean particle size of 50 nm to 150 µm.

12. The method of therapy and/or prevention for a disease of a tissue in the posterior segment according to claim 10 wherein the fine particle is made of a biodegradable or biosoluble polymer.

13. The method of therapy and/or prevention for a disease of a tissue in the posterior segment according to claim 10 wherein the tissue in the posterior segment of the eye is retina, choroid or an optic nerves.

14. The method of therapy and/or prevention for a disease of a tissue in the posterior segment of the eye according to claim 10 wherein the drug is for use in therapy and/or prevention for a retinal, choroidal or optic nerve disease.
